# EUROPÄISCHE PATENTANMELDUNG

(11) **EP 1 925 321 A1**
(43) Veröffentlichungstag der Anmeldung: **28.05.2008**
(21) Anmeldenummer: 06090205.3
(22) Anmeldetag: 09.11.2006
(51) Int. Cl.: A61L 2/18, A61L 2/24, A61L 2/02

(54) **Reinigungsvorrichtung und -verfahren für medizinische Rohrschaft- und Hohlinstrumente**

(71) Anmelder: BANDELIN electronic GmbH & Co. KG, 12207 Berlin (DE)
(72) Erfinder: Ceolin, Peter, 10318 Berlin (DE); Gebauer, Dirk, 12249 Berlin (DE); Hagelstein, Erik, 81249 München (DE); Jung, Rainer, 13125 Berlin (DE); Kähler, Sebastian, 13187 Berlin (DE); Bandelin, Stefan, 14129 Berlin (DE)
(74) Vertreter: Pfenning, Meinig & Partner GbR

(57) **Zusammenfassung**

Die Erfindung betrifft eine Reinigungsvorrichtung zum Reinigen von mehreren spülbaren medizinischen Instrumenten, ein Verfahren zum Betreiben einer derartigen Vorrichtung, sowie ein Verfahren zum Reinigen von mehreren spülbaren medizinischen Instrumenten.

Die erfindungsgemäße Reinigungsvorrichtung (1) zur Reinigung von spülbaren medizinischen Instrumenten (11), insbesondere Rohrschaftinstrumenten oder Hohlinstrumenten, enthält eine Aufnahmeform (2) für die Aufnahme von Reinigungsflüssigkeit und für die Aufnahme von Instrumenten, mindestens einen von der Aufnahmeform (2) aus zugänglichen ersten (3) und zweiten Druckanschluss (4) für den Anschluss jeweils eines Instrumentes, einen Kanalwähler (13), eine Kanalzusammenführung (5), einen Durchflussmesser (6), eine Druckvorrichtung (10), sowie einen ersten Einzelkanal (7), der vom ersten Druckanschluss (3) über den Kanalwähler (13) zur Kanalzusammenführung (5) verläuft, einen zweiten Einzelkanal (8), der vom zweiten Druckanschluss (4) über den Kanalwähler (13) zur Kanalzusammenführung (5) verläuft, und einen Hauptkanal (9), der von der Kanalzusammenführung (5) zur Druckvorrichtung (10) und über den Durchflussmesser (6) verläuft, wobei der Kanalwähler (13) derart ausgebildet ist, dass durch diesen ein Einzelkanal (7) geöffnet werden kann und gleichzeitig sämtliche andere Einzelkanäle (8) schließbar sind, die Kanalzusammenführung (5) den ersten (7) und zweiten Einzelkanal (8) in den Hauptkanal (9) zusammenführt, mittels dem Durchflussmesser (6) der Durchfluss von Flüssigkeit im Hauptkanal (9) messbar ist, und mittels der Druckvorrichtung (10) über Hauptkanal (9) und ersten (7) und zweiten Einzelkanal (8) an den Druckanschlüssen (3, 4) ein Druck anlegbar ist, so dass mittels der Druckvorrichtung (10) durch die jeweils an einen Druckanschluss (3) mit einem Ende angeschlossenen Rohrschaft- oder Hohlinstrumente (11) eine Flüssigkeit drückbar ist und der Durchfluss der Flüssigkeit mittels Durchflussmesser (6) messbar ist.

## Beschreibung

Die Erfindung betrifft eine Reinigungsvorrichtung für mehrere medizinische Rohrschaftinstrumente oder Hohlinstrumente, ein Verfahren zum Betreiben einer derartigen Reinigungsvorrichtung und ein Verfahren zur Reinigung mehrerer medizinischer Rohrschaft- oder Hohlinstrumente.

Im Bereich der minimalinvasiven Chirurgie (MIC) werden sogenannte Rohrschaftinstrumente oder Hohlinstrumente verwendet, die sich dadurch auszeichnen, dass diese einen langen, dünnen, schaftförmigen Abschnitt aufweisen, in denen beispielsweise Zugstangen, Kabel oder ähnliches untergebracht sind. Durch den Gebrauch werden die Instrumente verschmutzt, weswegen eine nachfolgende Reinigung notwendig ist. Insbesondere kann auch Schmutz zumindest in den Endbereich des schaftförmigen Abschnitts des Instrumentes eindringen, was die Reinigung erheblich kompliziert.

Aus der DE 44 41 401 A1 ist eine Reinigungsvorrichtung für medizinische Rohrschaftinstrumente oder Hohlinstrumente bekannt, die eine als Wanne ausgebildete Aufnahmeform für die Aufnahme von Reinigungsflüssigkeit und für die Aufnahme mehrerer Instrumente, einen Adapter für die Aufnahme eines Endes jeweils eines Instrumentes, einen mit dem Adapter verbundenen Sauganschluss und eine Pumpe als Saugvorrichtung aufweist. Mittels der Saugvorrichtung ist es möglich, sich in der Aufnahmeform befindende Reinigungsflüssigkeit durch den Hohlraum des medizinischen Instrumentes zu saugen und damit das Instrument zu reinigen. Der Adapter dichtet dabei das eine Ende des Hohlraums gegen den Außenraum ab, damit verhindert wird, dass die Flüssigkeit direkt aus dem Bad angesaugt wird, ohne das Instrument zu passieren. Über einen Einlauf wird die angesaugte Flüssigkeit dem Bad wieder zugeführt. Des Weiteren weist die Reinigungsvorrichtung eine Ultraschallvorrichtung auf, mit der die Reinigungsflüssigkeit in der Aufnahmeform mit Ultraschall beaufschlagt werden kann.

Eine ähnliche Reinigungsvorrichtung offenbart die Druckschrift DE 92 08 607 U1.

Nachteil dieser Reinigungsvorrichtungen ist, dass, wenn mehrere Instrumente gereinigt werden sollen, zeitgleich an mehreren Instrumenten gesaugt wird. Es ist hierbei möglich, dass Reinigungsflüssigkeit in unterschiedlichen Mengen durch die Instrumente hindurchtritt und der Reinigungseffekt stark unterschiedlich und bei manchen Instrumenten sogar ungenügend ist. Alternativ schlägt beispielsweise die DE 44 41 401 A1 einkanalige Pumpen oder mehrkanalige Pumpen vor. Hier ist allerdings der Nachteil, dass ein solches System sehr aufwendig ist.

Ein weiterer Nachteil des Stands der Technik ist, dass diese Reinigungsvorrichtungen keine Möglichkeit vorsehen, die es ermöglichen, zu beurteilen, ob die Instrumente auch tatsächlich genügend gereinigt wurden. In der DE 44 41 401 A1 kann man zwar anhand des Austritts von Reinigungsflüssigkeit am Einlauf erkennen, ob Flüssigkeit durch ein Instrument gesaugt wurde, dies lässt aber keine Rückschlüsse auf die Qualität der Reinigung zu.

Aufgabe der vorliegenden Erfindung ist es somit, eine Reinigungsvorrichtung für die Reinigung von Rohrschaftinstrumenten oder Hohlinstrumenten zu schaffen, die eine Reinigung von mehreren Instrumenten ermöglicht, die eine Aussage über die Qualität der Reinigung für jedes einzelne gereinigte Instrument liefert, und die kostengünstig und wartungsfreundlich mit einer geringen Anzahl von Komponenten aufbaubar ist. Eine weitere Aufgabe der Erfindung ist es, ein Verfahren zum Betreiben einer derartigen Reinigungsvorrichtung zu schaffen, sowie ein Verfahren zur Reinigung von mehreren Instrumenten zu schaffen.

Diese Aufgaben werden durch eine Reinigungsvorrichtung, ein Verfahren zum Betreiben einer Reinigungsvorrichtung und ein Verfahren zur Reinigung von mindestens zwei Instrumenten gelöst.

Vorteilhafte Weiterbildungen werden in den abhängigen Ansprüchen beschrieben.

Die Erfindung schafft eine zur Reinigung von medizinischen Rohrschaftinstrumenten oder Hohlinstrumenten geeignete Vorrichtung, enthaltend eine Aufnahmeform für die Aufnahme von Reinigungsflüssigkeit und für die Aufnahme von Instrumenten, mindestens einen von der Aufnahmeform aus zugänglichen ersten und zweiten Druckanschluss für den Anschluss jeweils eines Instrumentes, einen Kanalwähler, eine Kanalzusammenführung, einen Durchflussmesser, eine Druckvorrichtung, sowie einen ersten Einzelkanal, der vom ersten Druckanschluss über den Kanalwähler zur Kanalzusammenführung verläuft, einen zweiten Einzelkanal, der vom zweiten Druckanschluss über den Kanalwähler zur Kanalzusammenführung verläuft, und einen Hauptkanal, der von der Kanalzusammenführung zur Druckvorrichtung und über den Durchflussmesser verläuft, wobei der Kanalwähler derart ausgebildet ist, dass durch diesen ein Einzelkanal geöffnet werden kann und gleichzeitig sämtliche andere Einzelkanäle schließbar sind, die Kanalzusammenführung den ersten und zweiten Einzelkanal in den Hauptkanal zusammenführt, mittels dem Durchflussmesser der Durchfluss von Flüssigkeit im Hauptkanal messbar ist, und mittels der Druckvorrichtung über Hauptkanal und ersten und zweiten Einzelkanal an den Druckanschlüssen ein Druck anlegbar ist, so dass mittels der Druckvorrichtung durch die jeweils an einen Druckanschluss mit einem Ende angeschlossenen Rohrschaft- oder Hohlinstrumente eine Flüssigkeit drückbar ist und der Durchfluss der Flüssigkeit mittels Durchflussmesser messbar ist.

Eine Reinigung mit der erfindungsgemäßen Reinigungsvorrichtung beinhaltet insbesondere eine Reinigung der Instrumente von Schmutz und/oder Desinfektion der Instrumente.

Die erfindungsgemäße Reinigungsvorrichtung lässt sich insbesondere gemäß einem Verfahren mit folgenden Schritten betreiben: a) Anschließen jeweils eines Rohrschaftinstrumentes oder Hohlinstrumentes mit einem Ende an einen Druckanschluss und Eintauchen des anderen Endes in eine sich in der Aufnahmeform befindende Reinigungsflüssigkeit; b) Nacheinander öffnen jeweils eines einzelnen Einzelkanals und verschließen oder geschlossen halten der übrigen Einzelkanäle mittels des Kanalwählers und Saugen oder Pressen von Reinigungsflüssigkeit mittels der Druckvorrichtung durch das an den jeweils offenen Einzelkanal angeschlossene Rohrschaftinstrument; c) Messen des Durchflusses der durch ein einzelnes Rohrschaftinstrument gesaugten Reinigungsflüssigkeit mittels des Durchflussmessers.

Die erfindungsgemäße Reinigungsvorrichtung erlaubt es somit, bei der Reinigung von mehreren Instrumenten festzustellen, ob und wieviel Reinigungsflüssigkeit durch das jeweilige Instrument gespült wurde und erlaubt damit eine Aussage, ob das Instrument ausreichend gespült worden ist oder ob die Spülung unzureichend war. Dies wird dadurch ermöglicht, dass jeweils nur ein Instrument gespült und der Durchfluss der Reinigungsflüssigkeit durch allein dieses Instrument gemessen wird.

Dadurch, dass die verschiedenen Einzelkanäle von der Kanalzusammenführung zu einem Hauptkanal zusammengeführt werden, sind nur eine einzelne einkanalige Druckvorrichtung und ein einzelner einkanaliger Durchflussmesser notwendig. Insbesondere können für Druckvorrichtung und Durchflussmesser handelsübliche Durchflussmesser und Druckvorrichtungen eingesetzt werden. Aufgrund dessen, dass nur wenige und handelsübliche Komponenten benötigt werden, ist die Reinigungsvorrichtung kostengünstig herstellbar und auch wartungsfreundlich.

Vorzugsweise wird beim Reinigen eines Instrumentes zumindest das Unterschreiten eines mit dem Durchflussmesser gemessenen Mindestdurchflusswertes angezeigt (beispielsweise über ein Display) und/oder aufgezeichnet (beispielsweise auf einem Speichermedium). Besonders bevorzugt wird direkt der Messwert des Durchflussmessers während der Reinigung angezeigt und/oder aufgezeichnet. In beiden Fällen kann dies mit einer Fehlermeldung gekoppelt werden, die dem Benutzer schnell erkennen lässt, ob die Reinigung eines Instrumentes erfolgreich war oder nicht.

Die Aufnahmeform ist vorzugsweise als Wanne ausgebildet, in die die zu reinigenden Instrumente vollständig in eine sich in der Wanne befindenden Reinigungsflüssigkeit eingetaucht werden können.

Vorzugsweise ist der Durchflussmesser in Flussrichtung der gepumpten Reinigungsflüssigkeit hinter der Druckvorrichtung angeordnet, d.h., die Flüssigkeit passiert zunächst die Druckvorrichtung und dann den Durchflussmesser. Grundsätzlich ist es natürlich möglich, den Durchflussmesser auch an anderen Stellen des Hauptkanals anzuordnen.

Die Reinigungsvorrichtung ist selbstverständlich nicht auf die Reinigung von nur zwei Instrumenten beschränkt, sie kann für die Reinigung einer beliebigen Anzahl von Instrumenten ausgebildet sein, Die Anzahl der Anschlüsse und Einzelkanäle erhöht sich entsprechend.

Die Druckvorrichtung ist vorzugsweise als eine Vorrichtung zum Saugen von Flüssigkeit und/oder als eine Vorrichtung zum Pressen von Flüssigkeit ausgebildet und derart angeordnet, dass mittels der Druckvorrichtung durch die jeweils an einen Druckanschluss mit einem Ende angeschlossenen Instrumente eine Flüssigkeit gesaugt oder gepresst werden kann.

Vorzugsweise ist die Druckvorrichtung als Saugvorrichtung ausgebildet, dass heißt, die Reinigungsflüssigkeit wird nicht durch die Instrumente gepresst, sondern gesaugt. Dies hat den Vorteil, dass eine Verschmutzung des Instrumentes, die sich in der Regel am "Arbeitsende" des Instrumentes befindet, durch Anschließen dieses Endes an den Druckanschluss direkt abgesaugt werden kann, ohne dass der Schmutz durch den gesamten Hohlraum oder Rohrschaft des Instrumentes gepresst werden muss. Grundsätzlich ist allerdings auch ein Aufbau möglich, in dem Reinigungsflüssigkeit durch die Instrumente gepresst wird.

Sowohl für Pressen als auch Saugen lassen sich handelsübliche Pumpen als Druckvorrichtung einsetzen.

Eine vorteilhafte Weiterbildung der Erfindung sieht vor, dass die Reinigungsvorrichtung zusätzlich einen Einlauf aufweist, der sich im Bereich der Aufnahmeform befindet, und der Hauptkanal von der Druckvorrichtung zum Einlauf verläuft, zum Wiederzuleiten von angesaugter Flüssigkeit in die Aufnahmeform.

Hierdurch wird ein geschlossener Kreislauf der Reinigungsflüssigkeit realisiert. Ein Nachfüllen von Flüssigkeit während der Reinigung ist damit nicht notwendig.

Eine vorteilhafte Weiterbildung der Erfindung sieht vor, dass die Reinigungsvorrichtung zusätzlich einen Filter zum Filtern von Flüssigkeit aufweist, die durch den Hauptkanal fließt.

Durch den Filter wird die durch den Hauptkanal geführte Flüssigkeit gereinigt.

Vorzugsweise ist der Filter austauschbar.

Vorzugsweise ist der Filter in Flussrichtung der Flüssigkeit vor dem Durchflussmesser angeordnet, d.h., die Flüssigkeit passiert zunächst den Filter und dann den Durchflussmesser. Hierdurch wird ein schnelles Verschmutzen des Durchflussmessers verhindert.

Vorzugsweise ist der Filter in Flussrichtung der Flüssigkeit hinter der Druckvorrichtung angeordnet.

Eine vorteilhafte Weiterbildung der Erfindung sieht vor, dass erster Einzelkanal und zweiter Einzelkanal im Bereich des Kanalwählers geschlossenen sind, insbesondere keinen Kontakt mit mechanischen Teilen des Kanalwählers aufweisen.

"Geschlossen" soll in diesem Zusammenhang bedeuten, dass die Innenwandung des Einzelkanals durchgängig ist, insbesondere die Innenwandung nicht durch Teile von Dichtungen oder Ventilen durchbrochen ist. Ein derartiger geschlossener Abschnitt lässt sich beispielsweise durch einen einteiligen, in seiner Innenwandung geschlossenen Schlauch realisieren.

Eine vorteilhafte Weiterbildung der Erfindung sieht vor, dass der erste und zweite Einzelkanal zumindest im Bereich des Kanalwählers einen elastisch verformbaren Kanalabschnitt aufweisen, und der Kanalwähler eine Bewegungseinheit mit zumindest einem Quetschelement aufweist zum Quetschen und damit Verschließen eines jeweiligen Einzelkanals.

Durch das Quetschelement kann der jeweilige Einzelkanal zusammengequetscht werden, d.h., die Innenwände des Einzelkanals werden gegeneinandergedrückt, und damit ein Verschließen des Einzelkanals erreicht werden, ohne dass die Innenwandung des Kanals durchbrochen werden muss. Durch die Elastizität der Kanalabschnitte öffnen sich die Einzelkanäle selbständig beim Entfernen des Quetschelementes.

Vorzugsweise sind die Einzelkanäle schlauchförmig aus einem Elastomer, beispielsweise Silikon, ausgebildet.

Alternativ kann der Kanalwähler beispielsweise mit Ventilen, insbesondere mit Quetschventilen, mit pneumatisch beweglichen Elementen, mit Schiebern und/oder über Hähne realisiert sein, die sowohl manuell als auch automatisch bedienbar sein können.

Grundsätzlich ist es möglich, auch nichtelastische Materialien für derartige Kanalabschnitte einzusetzen. In diesem Falle wird das Öffnen der Kanäle vorzugsweise mechanisch unterstützt.

Eine vorteilhafte Weiterbildung der Erfindung sieht vor, dass der Hauptkanal im Bereich der Druckvorrichtung und/oder Durchflussmesser geschlossen ist, insbesondere keinen Kontakt mit mechanischen Teilen der Druckvorrichtung bzw. des Durchflussmessers aufweist.

Die Druckvorrichtung ist in diesem Sinne vorzugsweise als Schlauchpumpe bzw. Peristaltikpumpe ausgebildet.

Die Durchflussmessung ist hierfür über eine kalorimetrische, induktive oder Ultraschallmessung möglich.

Vorzugsweise ist der Hauptkanal schlauchförmig aus einem Elastomer, beispielsweise Silikon, ausgebildet.

Eine vorteilhafte Weiterbildung der Erfindung sieht vor, dass die Reinigungsvorrichtung einen ersten und ein zweiten Adapter mit einem Hohlraum aufweist, wobei der erste und zweite Adapter jeweils einen Adapteranschluss aufweist, über den der Hohlraum des ersten Adapters mit dem ersten Druckanschluss verbunden ist, und der Hohlraum des zweiten Adapters mit dem zweiten Druckanschluss verbunden ist, und der erste und zweite Adapter jeweils eine abdichtbare Durchführung aufweist, durch die ein Ende eines Rohrschaftinstrumentes in den Hohlraum des Adapters einführbar und gegenüber dem Außenraum des Adapters gegen Flüssigkeit abdichtbar ist.

Ein derartiger Adapter ermöglicht ein einfaches Anschließen jeweils eines Instrumentes an einen Druckanschluss. Durch die Abdichtung gegenüber dem Außenraum wird verhindert, dass insbesondere Flüssigkeit an dem Instrument vorbei gesaugt oder gepresst wird, was die Effektivität der Reinigung vermindern würde.

Die Durchführung kann insbesondere mit einem flexiblen Öffnungsquerschnitt ausgebildet sein, so dass Instrumente mit unterschiedlichen Außendurchmessern flüssigkeitsdicht abgedichtet werden können. Ein derartiger variabler Öffnungsquerschnitt lässt sich beispielsweise mit einer elastischen Membran herstellen.

Eine vorteilhafte Weiterbildung der Erfindung sieht vor, dass die Reinigungsvorrichtung zusätzlich eine Vorrichtung zum Erzeugen von Ultraschall im Bereich der Aufnahmeform aufweist.

Eine sich in der Aufnahmeform befindende Flüssigkeit kann auf diese Weise mit Ultraschall beaufschlagt werden, um die Wirkung der Reinigung zu verstärken.

Vorzugsweise wird der Hohlraum des jeweiligen Adapters, in den ein zu reinigendes Instrument eingeführt ist, ausreichend mit Flüssigkeit gefüllt, um den Ultraschall effektiv an das in der Regel besonders verschmutzte Ende des Instrumentes zu leiten.

Eine vorteilhafte Weiterbildung der Erfindung sieht vor, dass die Reinigungsvorrichtung eine Steuerelektronik aufweist, mittels der zumindest Kanalwähler, Durchflussmesser und Druckvorrichtung automatisch steuerbar sind.

Mit einer derartigen Steuervorrichtung können insbesondere ablaufende Programme zur Reinigung von mehreren Instrumenten realisiert werden, insbesondere oben beschriebene Verfahren automatisiert werden. Die Steuervorrichtung weist vorzugsweise Anzeige- und Bedienelemente auf, über die beispielsweise Parameter (wie Dauer der Reinigung, Temperatur der Reinigungsflüssigkeit oder Ultraschall) einstellbar sind und über die der Status des Reinigungsvorgangs ablesbar ist, insbesondere welche Instrumente nicht gereinigt werden konnten.

Die Erfindung wird nun anhand eines Ausführungsbeispiels, welches durch mehrere Figuren dargestellt wird, näher erläutert.

Dabei zeigt:
- Figur 1: eine Seitenansicht einer erfindungsgemäßen Reinigungsvorrichtung,
- Figur 2: eine Draufsicht der Reinigungsvorrichtung,
- Figur 3: eine Innenansicht mit elektronischen und mechanischen Komponenten und Kanalführung der Reinigungsvorrichtung,
- Figur 4: eine Prinzipdarstellung des Aufbaus der Reinigungsvorrichtung,
- Figuren 5a, 5b und 5c: mehrere Alternativen zur Führung der Reinigungsflüssigkeit,
- Figur 6: eine Explosionsdarstellung eines erfindungsgemäßen Kanalwählers,
- Figur 7: einen Querschnitt des Kanalwählers,
- Figur 8: eine Frontansicht des Kanalwählers,
- Figur 9: eine Explosionsdarstellung eines erfindungsgemäßen Adapters,
- Figur 10: ein Ablaufschema für die Durchführung einer Reinigung mit der erfindungsgemäßen Reinigungsvorrichtung mit mehreren Reinigungszyklen, und
- Figur 11: ein Ablaufschema für einen einzigen Reinigungszyklus.

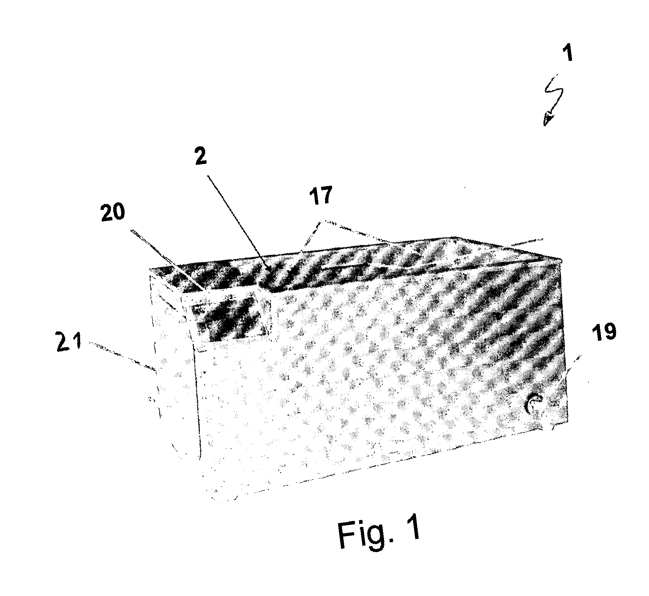

Die Figur 1 zeigt eine Seitenansicht einer erfindungsgemäßen Reinigungsvorrichtung 1.

Die Reinigungsvorrichtung beinhaltet eine offene, als Wanne ausgebildete Aufnahmeform 2 für die Aufnahme mehrerer, hier 12, zu reinigender medizinischer Rohrschaft- oder Hohlinstrumente und für die Aufnahme von Reinigungsflüssigkeit. Die Wanne 2 weist Vorsprünge 17 auf, auf die insbesondere ein Siebkorb 18 aufgelegt werden kann. Ein Ablauf 19 ist am Boden der Reinigungsvorrichtung vorgesehen, über den sich die Reinigungsflüssigkeit aus der Wanne entfernen lässt. Über eine Wartungsöffnung 21 im Außengehäuse der Reinigungsvorrichtung sind elektronische sowie mechanische Komponenten und das Kanalsystem zur Führung der Flüssigkeit der Reinigungsvorrichtung zugänglich.

Des Weiteren weist die Reinigungsvorrichtung ein druckempfindliches ("Touchscreen") kombiniertes Anzeige- und Bedienfeld 20 auf, über die sich die Reinigungsvorrichtung 1 steuern und der Status der Vorrichtung 1 ablesen lässt.

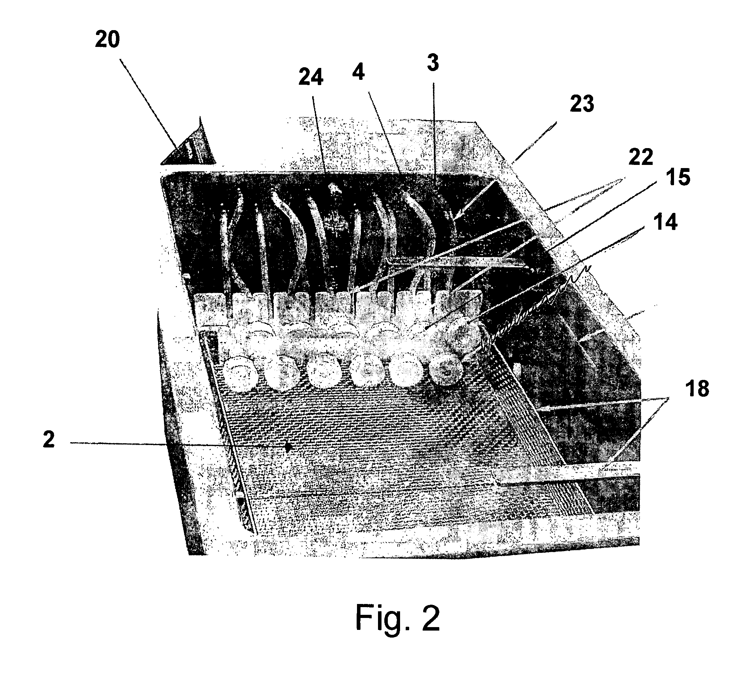

Figur 2 zeigt eine Draufsicht auf die erfindungsgemäße Reinigungsvorrichtung.

Die Reinigungsvorrichtung weist zwölf Druckanschlüsse, von denen ein erster und ein zweiter Druckanschluss exemplarisch mit 3 und 4 gekennzeichnet sind, auf, über die in diesem Falle ein Unterdruck angelegt werden kann und über die eine Reinigungsflüssigkeit ansaugbar ist. Die zwölf Druckanschlüsse sind gut zugänglich am oberen Rand der Wanne in zwei horizontal übereinander verlaufenden Reihen angeordnet. An jeweils einem Druckanschluss ist über einen Silikonschlauch ein Adapter angeschlossen, so z.B. an den ersten Druckanschluss 3 der Adapter 14 und an den zweiten Druckanschluss 4 der Adapter 15.

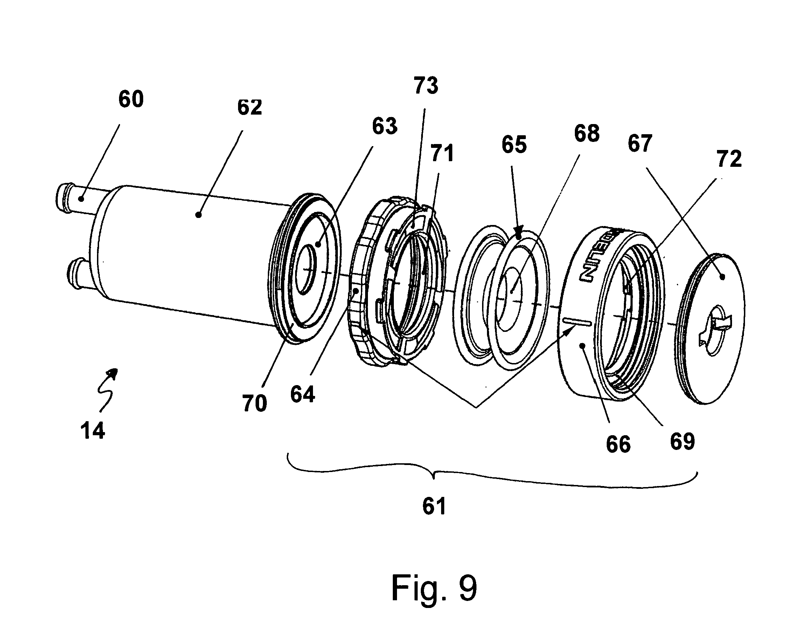

Die Adapter weisen einen hohlen, in Wasser durchsichtigen Behälter 62 auf (siehe hierzu Figur 9). Der Behälter weist einen Adapteranschluss 60 auf, an den der mit dem Druckanschluss verbundene Silikonschlauch angeschlossen ist und über den somit am Hohlraum ein Unterdruck anlegbar ist. Die Adapter weisen zu dem eine den Behälter 62 abschließende abdichtbare Durchführung 61 auf, durch die ein Ende eines Instrumentes 11 in den Hohlraum des Adapters einführbar und gegenüber dem Außenraum gegen Flüssigkeit abdichtbar ist.

Die Adapter sind in einer Halterung 22 in zwei horizontal übereinander liegenden Reihen an einem Ende eines Siebkorbes 18 angeordnet. Der restliche Platz des Siebkorbes steht der Aufnahme von zu reinigenden Instrumenten zur Verfügung. Über Haltegriffe kann der Siebkorb aus der Wanne 2 und einem sich darin befindenden Reinigungsbad gehoben werden, wodurch insbesondere der Anschluss von zu reinigenden Instrumenten an die jeweiligen Adapter und die spätere Entnahme der Instrumente erleichtert wird.

Des Weiteren weist die Reinigungsvorrichtung an der Innenwand der Wanne einen Füllstands- und Temperatursensor 23 zur Messung der Temperatur und des Füllstandes eines sich in der Wanne befindenden Bades auf.

Ebenfalls in der Innenwandung der Wanne befindet sich ein, in der Figur nicht sichtbarer, Einlauf 12, durch den über die Druckanschlüsse abgepumpte Flüssigkeit wieder in die Wanne 2 geleitet wird.

Die über die Sauganschlüsse angesaugte Flüssigkeit wird, bevor sie wieder in die Wanne gelangt, zur Reinigung über einen Filter 24 geleitet. Der Filter 24 ist dabei als auswechselbares Element ausgebildet und an der Innenwand der Wanne 2 angeordnet, kann somit einfach von Hand ausgetauscht werden.

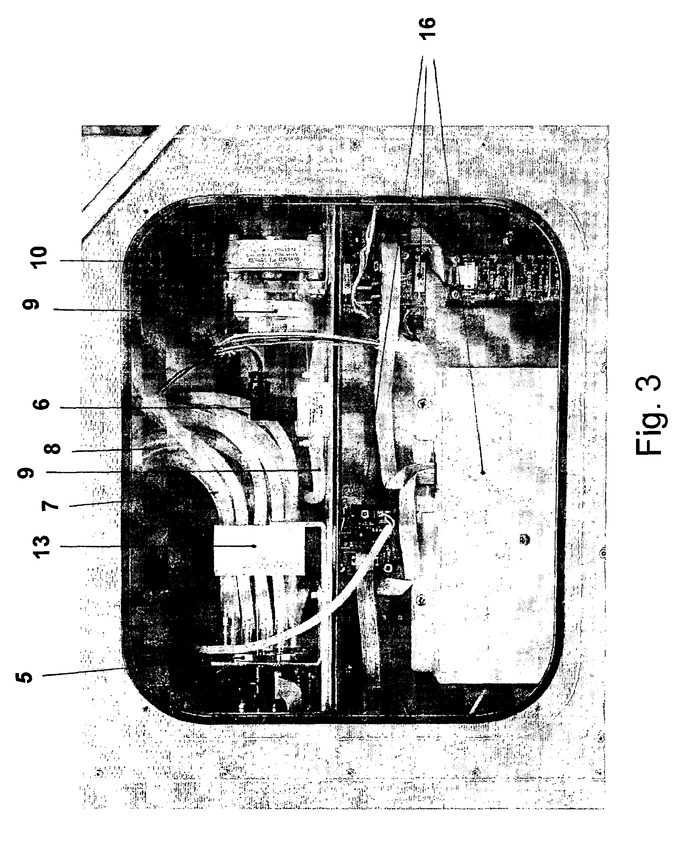

Figur 3 zeigt eine Innenansicht mit elektronischen und mechanischen Komponenten der Reinigungsvorrichtung bei geöffneter Wartungsöffnung 21.

Im über die Wartungsöffnung 21 zugänglichen Innenraum der Reinigungsvorrichtung befindet sich ein Kanalwähler 13, eine Kanalzusammenführung 5, ein Durchflussmesser 6, eine Druckvorrichtung 10, hier eine Peristaltikpumpe 10, sowie eine Steuerelektronik 16 zur Steuerung des Kanalwählers 13, des Durchflussmessers 6 und der Pumpe 10.

Des Weiteren weist die Reinigungsvorrichtung 12 Einzelkanäle (zwei Einzelkanäle sind exemplarisch mit 7 und 8 gekennzeichnet) und einen Hauptkanal 9 auf. Jeweils ein Einzelkanal wird durch einen einstückigen, Silikonschlauch mit einer geschlossenen Wandung gebildet und verläuft ununterbrochen von einem Druckanschluss über den Kanalwähler 13 zur Kanalzusammenführung 5. Der Kanalwähler ist dabei derart ausgebildet, dass durch diesen ein Einzelkanal geöffnet werden kann und gleichzeitig sämtliche andere Einzelkanäle geschlossen sind.

Die Kanalzusammenführung 5 ist als Ringkanal mit 13 Anschlussstutzen ausgebildet. An 12 Anschlussstutzen sind die 12 Einzelkanäle aufgesteckt, an den 13. Anschlussstutzen der Hauptkanal 9, der hier ebenfalls von einem Silikonschlauch gebildet wird. Sämtliche Einzelkanäle münden somit in den Hauptkanal 9.

Der Hauptkanal 9 verläuft als ununterbrocher Silikonschlauch über die Schlauchpumpe 10 zum Filter 24 (siehe Figur 2). Nach Passieren des Filters 24 führt der Hauptkanal 9 über einen weiteren Silikonschlauch zum Durchflussmesser 6 und von da aus zum Einlauf 12.

Aufgrund dessen, dass die Einzelkanäle im Bereich des Kanalwählers 13 und der Hauptkanal im Bereich der Pumpe 10 geschlossen sind, besteht keine Möglichkeit, dass in den Kanälen geführte Flüssigkeit mit mechanischen Teilen dieser Komponenten in Kontakt kommt. Zu dem werden Stellen vermieden, an denen sich Schmutz anlagern kann. Auch falls die Kanäle verschmutzt oder beschädigt sein sollten, ist ein einfacher, kostengünstiger Austausch möglich, da die Kanäle von genannten Komponenten getrennt sind.

Der Durchflussmesser kann auch für eine kalorimetrische, induktive oder Ultraschallmessung ausgebildet sein, so dass auch in diesem Bereich der Hauptkanal wie beschrieben geschlossen sein kann.

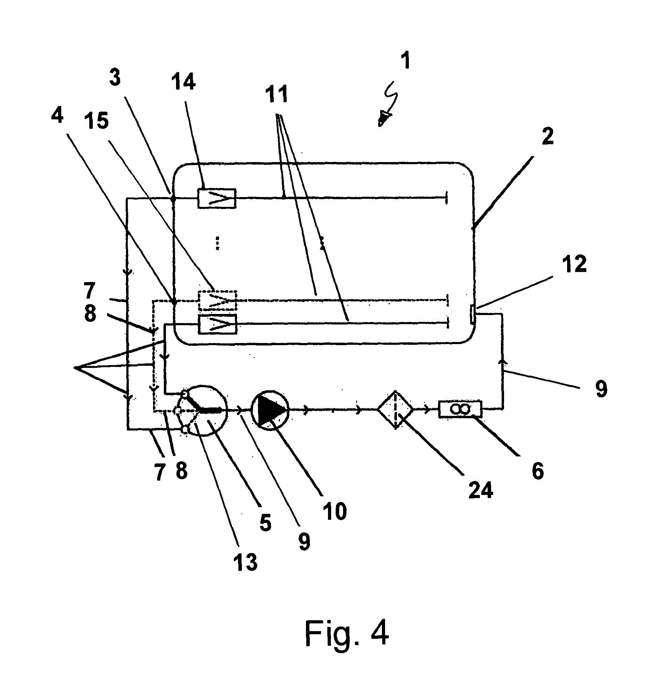

Figur 4 zeigt den beschriebenen Aufbau und den Verlauf der Kanäle in einer Prinzipdarstellung. Des Weiteren zeigt Figur 4 Instrumente 11, die in die Adapter eingeführt sind.

Die Reinigunsvorrichtung 1 weist zu dem eine (hier nicht gezeigte) Ultraschallvorrichtung auf, die im Boden der Aufnahmeform 2 angeordnet ist, und über die ein sich in der Aufnahmeform 2 befindendes Flüssigkeitsbad mit Ultraschall beaufschlagt werden kann.

Kanalwähler 13, Durchflussmessers 6, Pumpe 10, Temperatur- und Füllstandssensors 23 und Ultraschallvorrichtung sind mittels der Steuerelektronik 16 automatisch steuerbar bzw. auslesbar, wobei die Steuerelektronik über das Bedien- und Anzeigefeld 20 sowohl bedient als auch der Status der Geräte abgerufen und angezeigt werden kann.

Die hier beschriebene Reinigungsvorrichtung 1 kann in einfacher Weise umgerüstet werden, so dass ein Pressen von Flüssigkeit durch die angeschlossenen Instrumente 11 möglich ist. Dies beinhaltet im Wesentlichen eine Änderung der Pumprichtung der Pumpe 10 und vorzugsweise auch ein Umsetzen des Durchflussmessers und des Filters 24 in Flussrichtung hinter die Pumpe 10.

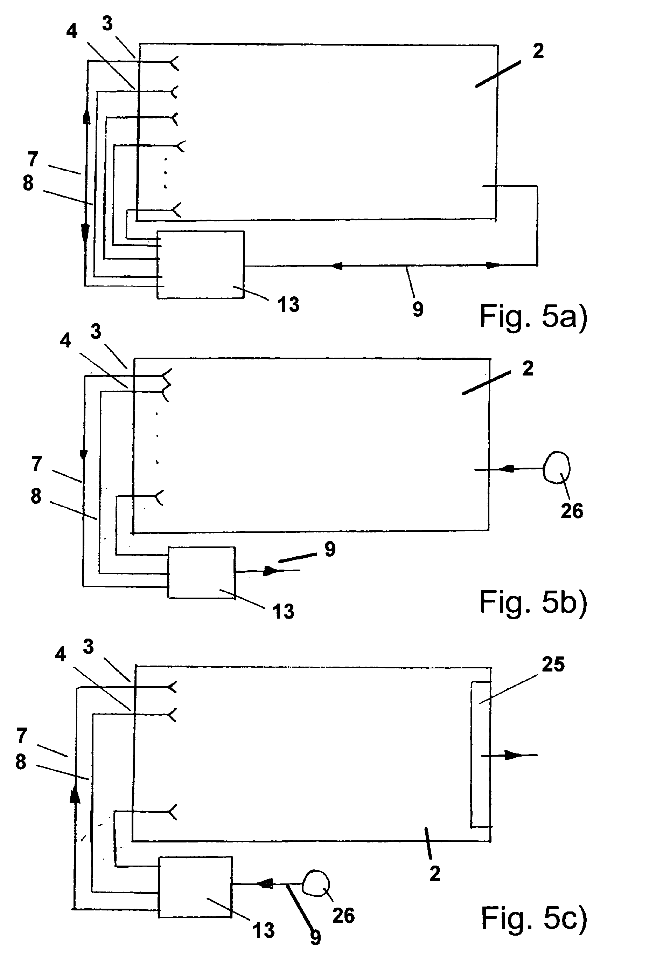

Die Figuren 5a, 5b und 5c zeigen schematisch verschiedene alternative Möglichkeiten zur Führung der Reinigungsflüssigkeit.

In einer ersten Alternative, siehe Figur 5a, ist ein geschlossener Flüssigkeitskreislauf realisiert. Die Flüssigkeit wird sowohl beim Pressen als auch beim Saugen, gekennzeichnet durch zwei Pfeile am hier exemplarisch offenen Einzelkanal 7 und Hauptkanal 9, dem sich in der Aufnahmeform 2 befindenden Reinigungsbad entnommen und wieder zugeführt.

In einer zweiten Alternative, siehe Figur 5b, wird die Reinigungsflüssigkeit beim Spülen der Instrumente aus dem sich in der Aufnahmeform 2 befindenden Reinigungsbad gesaugt und in einen hier nicht näher dargestellten Behälter gepumpt, also dem Bad nicht wieder zugeführt. In der Figur ist die Flussrichtung der Flüssigkeit durch die Pfeile am Einzelkanal 7 und Hauptkanal 9 dargestellt. Ein zusätzlicher Vorratsbehälter 26 gewährleistet hier das Nachfließen von Reinigungsflüssigkeit in die Aufnahmeform 2.

In einer dritten Alternative, siehe Figur 5c, wird die Reinigungsflüssigkeit dem Vorratsbehälter 26 entnommen und durch Pressen durch die Instrumente in die Aufnahmeform 2 geleitet, wie durch die Pfeile am Einzelkanal 7 und Hauptkanal 9 dargestellt. Ein Überlauf 25 gewährleistet ein kontrolliertes Abfließen von Flüssigkeit aus der Aufnahmeform 2, insbesondere dass ein konstanter Flüssigkeitspegel in der Aufnahmeform 2 aufrecht erhalten werden kann.

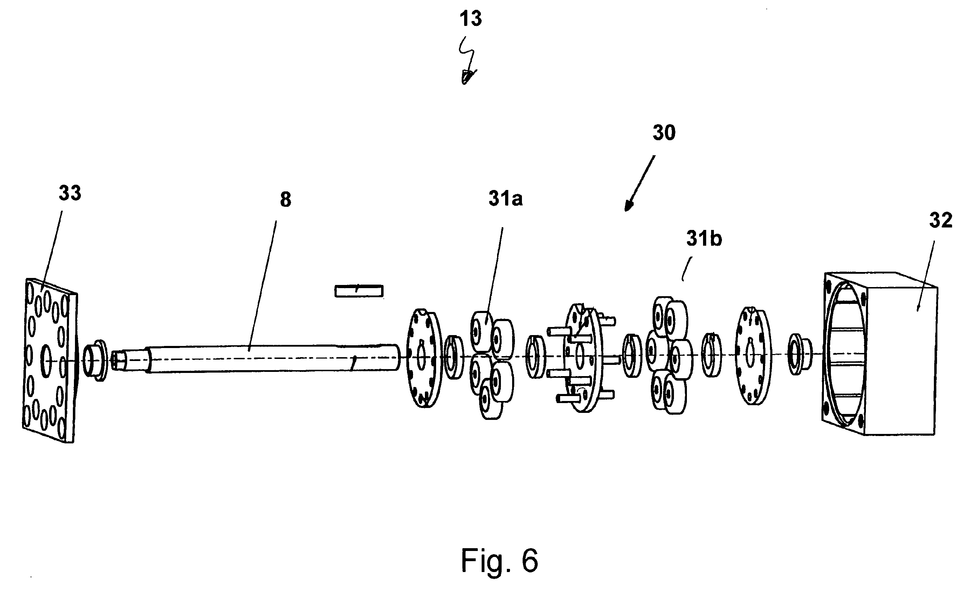

Die Figuren 6 bis 8 zeigen den Kanalwähler in verschiedenen Ansichten.

Der Kanalwähler 13 weist eine Bewegungseinheit 30 mit elf Quetschelementen 31a und 31b auf zum Quetschen und damit Verschließen eines jeweiligen Einzelkanals.

Die Quetschelemente sind derart angeordnet, dass jeweils elf Einzelkanäle, beispielsweise auch Einzelkanal 8, dicht gegen eine gegenüberliegende Wand, gebildet durch die die Quetschelemente 31 umschließende Innenwand eines massiven Elementes 32, zusammengequetscht werden, ein Einzelkanal, beispielsweise Einzelkanal 7, jedoch nicht gequetscht wird.

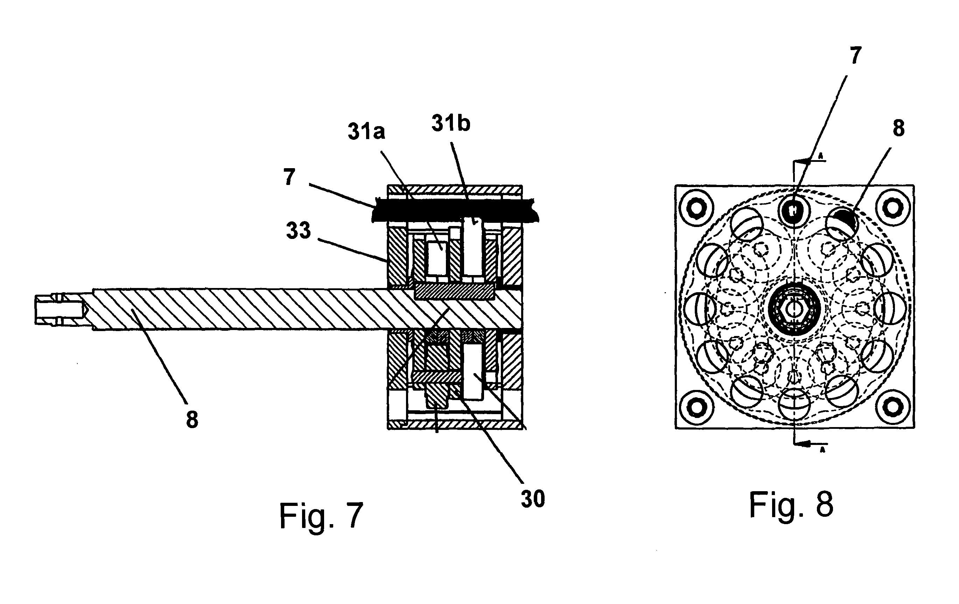

In diesem Ausführungsbeispiel sind die Quetschelemente 31a, 31b als Rollen aus einem Hartgummi ausgebildet und zu fünft (31a) und zu sechst (31b) in zwei zueinander parallelen Ebenen kreisförmig mit identischen Winkelabständen zwischen den jeweiligen Rollen drehbar gelagert angeordnet, allerdings von Ebene zu Ebene um eine Hälfte des Winkelabstandes versetzt, siehe insbesondere Figur 7. Dies hat zur Folge, dass eine Lücke vorhanden ist. Ein Einzelkanal, der sich in dieser Lücke befindet, wird somit nicht gequetscht.

Kreisförmig um die Rollen 31a, 31b herum und von einer Aufnahme 33 und dem massiven Element 32 gehalten sind jeweils um eine Hälfte des obigen Winkelabstandes der Rollen versetzt die Schläuche der Einzelkanäle angeordnet.

Die Rollen 31a, 31b sind mit einer Achse 8 verbunden, so dass diese durch Drehen der Achse 8 um diese Achse rotieren. Die Achse 8 ist selbst mit einem Elektromotor verbunden und darüber drehbar. Der Motor ist hier allerdings nicht näher dargestellt.

Über Drehen der Achse bewegen sich die Rollen 31a, 31b gegenüber den örtlich festen Einzelkanälen. Durch die symmetrische Anordnung kann auf dieses Weise die Lücke in der Anordnung der Quetschelemente 31 auf jeden der zwölf Einzelkanäle gebracht werden, so dass dieser geöffnet ist. Gleichzeitig werden die übrigen elf Einzelkanäle durch die Quetschelemente verschlossen.

Figur 9 zeigt eine Explosionsdarstellung eines erfindungsgemäßen Adapters 14.

Der Adapter weist, wie oben schon kurz beschrieben, einen Behälter 62 und eine abdichtbare Durchführung 61, die den Deckel des Behälters 62 bildet, auf.

Die abdichtbare Durchführung setzt sich in diesem Ausführungsbeispiel aus einer Lochscheibe 63, einem Haltering 64, einer Dichtung 65, einem Drehring 66 und einer Klemmscheibe 67 zusammen.

Die Dichtung 65 ist als nach innen gewölbter Ring mit geschlossener Fläche und einer Öffnung 68 im Zentrum ausgebildet und besteht aus einem hochelastischen Material, hier ein spezieller additionsvernetzter Silikonkautschuk.

Die Dichtung 65 ist an einem ihrer wulstförmig ausgebildeten Außenränder über den gesamten Umfang des Randes fest mit dem Drehring 66 verbunden. Diese Verbindung ist dadurch hergestellt, dass der Rand der Dichtung 65 zwischen einem innen liegenden umlaufenden Steg 69 des Drehrings 66 und der in den Drehring geschraubten Klemmscheibe 67 umlaufend eingeklemmt ist.

Die Dichtung 65 ist mit ihrem zweiten Außenrand über den gesamten Umfang des Randes fest mit dem Haltering 64 verbunden. Der Rand der Dichtung 65 liegt dabei auf einem flanschförmigen Rand 70 der Öffnung des Behälters 62 auf und wird zwischen diesem und von einem sich auf der Innenwand des Halterings 64 befindenden umlaufenden Steg 71 bei auf dem Behälter aufgeschraubten Haltering 64 umlaufend eingeklemmt.

Eine genügend große Öffnung in der Klemmscheibe 67 und in der Lochscheibe 63 gewährleistet, dass Instrumente mit unterschiedlichen Außendurchmessern, hier bis zu 10mm, durch die Öffnung der Dichtung 65 in den Innenraum des Behälters 62 eingeführt werden können.

Durch Drehen des Drehrings gegen den Haltering schnürt sich die Dichtung 65, die ja sowohl von Haltering und Drehring an jeweils einem ihrer beiden Außenrändern fest gehalten wird, zusammen, der Öffnungsquerschnitt der Öffnung 68 verkleinert sich. Insbesondere legt sich die Dichtung 65 bei genügendem Drehen an die Außenfläche eines durch die Öffnung 68 der Dichtung geführten Instrumentes an und dichtet dieses somit gegen außen ab. Das Zusammenschnüren wird hier durch die Elastizität des Materials der Dichtung 65 ermöglicht, wobei die Elastizität gleichfalls für eine gute Dichtwirkung zwischen Dichtung 65 und Außenfläche des Instrumentes 11 sorgt.

Drehring und Haltering können mittels eines Rastmechanismus, in diesem Falle greifen Vorsprünge 72 des Drehrings 66 in Aussparungen 73 des Halterings 71 formschlüssig ein, in ihrer Stellung fixiert werden, so dass die zusammengeschnürte Dichtung 65 sich nicht ungewollt löst. Zum Lösen der Dichtung wird der Rastmechanismus aufgehoben, in diesem Fall in dem der Drehring leicht angehoben wird und damit die Vorsprünge des Drehringes 66 aus den Aussparungen des Halterings 71 gehoben werden, so dass der Formschluss aufgehoben ist.

Im Folgenden soll das Verfahren zur Reinigung von spülbaren, medizinischen Rohrschaft- oder Hohlinstrumenten, insbesondere Verfahren zum Betreiben der erfindungsgemäßen Reinigungsvorrichtung, näher beschrieben werden.

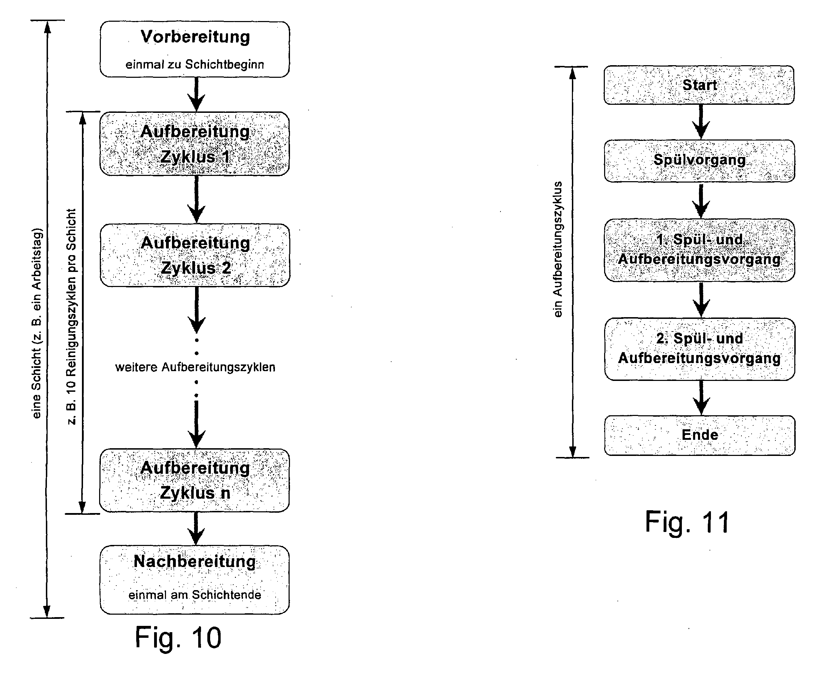

Figur 10 zeigt ein Ablaufschema für die Reinigung einer Vielzahl von derartigen Instrumenten in mehreren Aufbereitungszyklen/Reinigungszyklen.

In einem ersten Schritt ist die Reinigungsvorrichtung vorzubereiten. Dies umfasst das Einschalten der Vorrichtung, das Einfüllen von Reinigungsflüssigkeit und die Entgasung. Die Entgasung erfolgt hier automatisch durch ein in der Steuerelektronik vorhandenes Programm.

In einem zweiten Schritt wird ein Aufbereitungszyklus durchlaufen. Ein Aufbereitungszyklus umfasst dabei das Einlegen und Anschließen der Instrumente, den Start eines Reinigungsprogramms, und nach Beenden der Reinigung die Entnahme der Instrumente.

Je nach der Anzahl der zu reinigenden Instrumente können mehrere derartige Aufbereitungszyklen nacheinander ablaufen.

In einem dritten Schritt, nach dem alle vorgesehenen Aufbereitungszyklen durchgeführt wurden, wird eine Nachbereitung durchgeführt. Dies umfasst das Entleeren und Spülen der Reinigungsvorrichtung, möglicherweise das Wechseln des Filters 24, und das Ausschalten des Gerätes.

Der Ablauf eines einzelnen Aufbereitungszyklus ist in Figur 11 durch ein Schema näher dargestellt.

Ein Aufbereitungszyklus umfasst in diesem Ausführungsbeispiel folgende Schritte:

In einem ersten Schritt werden die Instrumente 11 an die Adapter bei aus dem sich in der Wanne 2 befindenden Reinigungsbad entnommenen Siebkorb 18 angeschlossen. Adapter und Instrumente werden dann durch Senken des Siebkorbes 18 in die Reinigungsflüssigkeit eingetaucht, so dass Flüssigkeit in die Instrumente eingesaugt werden kann.

In einem Spülvorgang wird Flüssigkeit in die Adapter gesaugt (Entlüftung) und es folgt eine zweite Entgasung. Nach der Entlüftung wird das Bad durch die Ultraschallvorrichtung mit Ultraschall beaufschlagt.

In einem ersten Spül- und Aufbereitungsvorgang werden die Instrumente nacheinander mit Reinigungsflüssigkeit durchspült, wobei gleichzeitig das Bad mit Ultraschall beaufschlagt wird. Hierfür wird jeweils mittels des Kanalwählers 13 ein einzelner Einzelkanal geöffnet und die übrigen Einzelkanäle verschlossen oder geschlossen gehalten. Dann wird eine Reinigungsflüssigkeit mittels der Druckvorrichtung 10 durch das an den jeweils offenen Einzelkanal angeschlossene Instrument gesaugt.

Es folgt ein zweiter Spül- und Aufbereitungsvorgang, der analog des ersten Vorganges durchgeführt wird.

Während der beiden Spül- und Aufbereitungsvorgänge wird der Durchfluss der durch ein einzelnes Instrument gesaugten Reinigungsflüssigkeit mittels des Durchflussmessers 6 gemessen und von der Steuerelektronik in einem Speicher aufgezeichnet und mittels eines Mikrocontrollers ausgewertet. Unterschreitet die während der Reinigung durch ein Instrument gesaugte Flüssigkeit einen Mindestwert, so erscheint auf dem Bedien- und Anzeigefeld 20 bei Beendigung der Spül- und Aufbereitungsvorgänge eine Fehlermeldung, die das oder die nichtgereinigten Instrumente durch Angabe des jeweiligen Druckanschluss bezeichnet. Sind alle Instrumente gereinigt, so wird die Reinigung ebenfalls durch eine Meldung auf dem Bedien- und Anzeigefeld als erfolgreich angezeigt.

In einem letzten Schritt werden die Instrumente entnommen. Es kann versucht werden, nicht gereinigte Instrumente in einem weiteren Zyklus zu reinigen.

Mittels der Steuerelektronik wird ein Aufbereitungszyklus, abgesehen von dem Anschluss und der Entnahme der Instrumente, automatisch durchgeführt. Entsprechende Reinigungsprogramme sind in der Steuerelektronik vorgesehen und können über das Bedienfeld 20 ausgewählt und gestartet werden. Insbesondere ist es möglich, Parameter wie Dauer der Reinigung, Temperatur des Bades, Ultraschall, Mindestdurchflusswerte einzustellen.

Mit der hier beschriebenen erfindungsgemäßen Reinigungsvorrichtung können bis zu zwölf spülbare, medizinische Hohlinstrumente oder Rohrschaftinstrumente mit Außendurchmessern von 1 bis 10mm in einer Wanne 2 gleichzeitig desinfiziert und gereinigt werden. Die spülbaren Instrumente werden dabei an jeweils einen zugeordneten Adapter angeschlossen, jeweils einzeln auf Durchlässigkeit geprüft und gespült und dabei desinfiziert und gereinigt. Das Durchspülen erfolgt in diesem Fall über einen Saugprozess am distalen Ende der Instrumente. Dass heißt, die Verschmutzungen werden immer entgegen der Richtung, in die sie eingedrungen sind, abgesaugt. Das restliche Lumen der Instrumente wird mit diesen Verschmutzungen nicht weiter verunreinigt. Das Dichtungsprinzip der Adapter gestattet das Anschließen von Instrumenten mit Durchmessern von 1 bis 10 mm, ohne dass zusätzliche Dichtungen notwendig sind. Nicht durchgängige Instrumente werden über die integrierte Durchlässigkeitsprüfung sicher identifiziert.

## Patentansprüche

1. Reinigungsvorrichtung (1) zur Reinigung von spülbaren medizinischen Instrumenten (11), insbesondere Rohrschaftinstrumenten oder Hohlinstrumenten, enthaltend eine Aufnahmeform (2) für die Aufnahme von Reinigungsflüssigkeit und für die Aufnahme von Instrumenten, mindestens einen von der Aufnahmeform (2) aus zugänglichen ersten (3) und zweiten Druckanschluss (4) für den Anschluss jeweils eines Instrumentes, einen Kanalwähler (13), eine Kanalzusammenführung (5), einen Durchflussmesser (6), eine Druckvorrichtung (10), sowie einen ersten Einzelkanal (7), der vom ersten Druckanschluss (3) über den Kanalwähler (13) zur Kanalzusammenführung (5) verläuft, einen zweiten Einzelkanal (8), der vom zweiten Druckanschluss (4) über den Kanalwähler (13) zur Kanalzusammenführung (5) verläuft, und einen Hauptkanal (9), der von der Kanalzusammenführung (5) zur Druckvorrichtung (10) und über den Durchflussmesser (6) verläuft, wobei der Kanalwähler (13) derart ausgebildet ist, dass durch diesen ein Einzelkanal (7) geöffnet werden kann und gleichzeitig sämtliche andere Einzelkanäle (8) schließbar sind, die Kanalzusammenführung (5) den ersten (7) und zweiten Einzelkanal (8) in den Hauptkanal (9) zusammenführt, mittels dem Durchflussmesser (6) der Durchfluss von Flüssigkeit im Hauptkanal (9) messbar ist, und mittels der Druckvorrichtung (10) über Hauptkanal (9) und ersten (7) und zweiten Einzelkanal (8) an den Druckanschlüssen (3, 4) ein Druck anlegbar ist, so dass mittels der Druckvorrichtung (10) durch die jeweils an einen Druckanschluss (3) mit einem Ende angeschlossenen Rohrschaft- oder Hohlinstrumente (11) eine Flüssigkeit drückbar ist und der Durchfluss der Flüssigkeit mittels Durchflussmesser (6) messbar ist.

2. Reinigungsvorrichtung (1) nach Anspruch 1, **dadurch gekennzeichnet, dass** die Druckvorrichtung (10) als eine Vorrichtung zum Saugen von Flüssigkeit und/oder als eine Vorrichtung zum Pressen von Flüssigkeit ausgebildet und derart angeordnet ist, dass mittels der Druckvorrichtung (10) durch die jeweils an einen Druckanschluss (3) mit einem Ende angeschlossenen Instrumente (11) eine Flüssigkeit gesaugt oder gepresst werden kann.

3. Reinigungsvorrichtung (1) nach einem der vorhergehenden Ansprüche, **dadurch gekennzeichnet, dass** die Reinigungsvorrichtung (1) zusätzlich einen Einlauf (12) aufweist, der sich im Bereich der Aufnahmeform (2) befindet, und der Hauptkanal (9) von der Druckvorrichtung (10) zum Einlauf (12) verläuft, zum Wiederzuleiten von angesaugter Flüssigkeit in die Aufnahmeform (2).

4. Reinigungsvorrichtung (1) nach einem der vorherigen Ansprüche, **dadurch gekennzeichnet, dass** die Reinigungsvorrichtung (1) zusätzlich einen Filter (24) zum Filtern von Flüssigkeit aufweist, die durch den Hauptkanal (9) fließt.

5. Reinigungsvorrichtung (1) nach einem der vorhergehenden Ansprüche, **dadurch gekennzeichnet, dass** erster Einzelkanal (7) und zweiter Einzelkanal (8) im Bereich des Kanalwählers (13) geschlossen sind, insbesondere keinen Kontakt mit mechanischen Teilen des Kanalwählers (13) aufweisen.

6. Reinigungsvorrichtung (1) nach Anspruch 5, **dadurch gekennzeichnet, dass** der erste und zweite Einzelkanal (7, 8) zumindest im Bereich des Kanalwählers (13) einen elastisch verformbaren Kanalabschnitt aufweisen, und der Kanalwähler (13) eine Bewegungseinheit (30) mit zumindest einem Quetschelement (31a, 31b) aufweist zum Quetschen und damit Verschließen eines jeweiligen Einzelkanals (7, 8).

7. Reinigungsvorrichtung (1) nach einem der vorhergehenden Ansprüchen, **dadurch gekennzeichnet, dass** der Hauptkanal (9) im Bereich der Druckvorrichtung (10) und/oder Durchflussmesser (6) geschlossen ist, insbesondere keinen Kontakt mit mechanischen Teilen der Druckvorrichtung (10) bzw. des Durchflussmessers (6) aufweist.

8. Reinigungsvorrichtung (1) nach einem der vorhergehenden Ansprüchen, **dadurch gekennzeichnet, dass** die Reinigungsvorrichtung (1) einen ersten (14) und einen zweiten Adapter (15) mit einem Hohlraum aufweist, wobei der erste und zweite Adapter (14, 15) jeweils einen Adapteranschluss aufweist (60), über den der Hohlraum des ersten Adapters (14) mit dem ersten Druckanschluss (3) verbunden ist, und der Hohlraum des zweiten Adapters (15) mit dem zweiten Druckanschluss (4) verbunden ist, und der erste und zweite Adapter (14, 15) jeweils eine abdichtbare Durchführung (61) aufweist, durch die ein Ende eines Rohrschaftinstrumentes in den Hohlraum des Adapters (14, 15) einführbar und gegenüber dem Außenraum des Adapters (14, 15) gegen Flüssigkeit abdichtbar ist.

9. Reinigungsvorrichtung (1) nach einem der vorhergehenden Ansprüche, **dadurch gekennzeichnet, dass** die Reinigungsvorrichtung (1) zusätzlich eine Vorrichtung zum Erzeugen von Ultraschall im Bereich der Aufnahmeform (2) aufweist.

10. Reinigungsvorrichtung (1) nach einem der vorhergehenden Ansprüche, **dadurch gekennzeichnet, dass** die Reinigungsvorrichtung (1) eine Steuerelektronik (16) aufweist, mittels der zumindest Kanalwähler (13), Durchflussmesser (6) und Druckvorrichtung (10) automatisch steuerbar sind.

11. Verfahren zum Betreiben einer Reinigungsvorrichtung (1) nach einem der Ansprüche 1 bis 10, enthaltend die Schritte: a) Anschließen jeweils eines spülbaren medizinischen Instrumentes (11) mit einem Ende an einen Druckanschluss (3) und Eintauchen des anderen Endes in eine sich in der Aufnahmeform (2) befindende Reinigungsflüssigkeit; b) Nacheinander öffnen jeweils eines einzelnen Einzelkanals (7, 8) und verschließen oder geschlossen halten der übrigen Einzelkanäle (7, 8) mittels des Kanalwählers (13) und Saugen oder Pressen von Reinigungsflüssigkeit mittels der Druckvorrichtung (10) durch das an den jeweils offenen Einzelkanal angeschlossene Rohrschaftinstrument; c) Messen des Durchflusses der durch ein einzelnes Rohrschaftinstrument (11) gesaugten oder gepressten Reinigungsflüssigkeit mittels des Durchflussmessers (6).

12. Verfahren zur Reinigung von zumindest zwei spülbaren medizinischen Instrumenten (11), insbesondere Rohrschaftinstrumenten oder Hohlinstrumenten, wobei nacheinander eine Reinigungsflüssigkeit jeweils ausschließlich nur durch ein einziges Instrument (11) gesaugt oder gepresst wird, und der Durchfluss der durch das jeweilige einzelne Instrument gesaugten oder gepressten Flüssigkeit gemessen wird.

13. Verfahren nach Anspruch 12, **dadurch gekennzeichnet, dass** das zumindest das Unterschreiten eines Mindestdurchflusswertes aufgezeichnet wird.
